# EUROPEAN PATENT SPECIFICATION

(11) **EP 0 970 099 B1**
(45) Date of publication and mention of the grant of the patent: **05.02.2003**
(21) Application number: 97907949.8
(22) Date of filing: 27.02.1997
(51) Int. Cl.: C07H 17/08

(54) **PROCESS FOR PREPARING ERYTHROMYCIN A OXIME**
VERFAHREN ZUR HERSTELLUNG VON ERYTHROMICIN A-OXIM
PROCEDE DE PREPARATION D'ERYTHROMYCINE A OXIME

(30) Priority: 10.04.1996 US 630306
(43) Date of publication of application: 12.01.2000
(73) Proprietor: Abbott Laboratories, Abbott Park, Illinois 60064-3500 (US)
(72) Inventor: CHANG, Sou-Jen, Prairie View, Illinois 60069 (US)
(74) Representative: Modiano, Guido, Dr.-Ing.
(86) International application number: PCT/US97/03177
(87) International publication number: WO 97/038000

(56) References cited:
- US-A- 3 478 014
- US-A- 4 150 220
- US-A- 4 526 889
- US-A- 5 274 085
- TETRAHEDR. LETT., vol. 2, 1970, pages 157-60, XP000654484 E.H. MASSEY ET. AL.: "Erythromycylamine"

## Description

### Technical Field Of The Invention

The field of the present invention is erythromycin A 9-oxime production. More particularly the present invention pertains to the production of 9-oxime erythromycin A using isopropanol and acetic acid.

### Background Of The Invention

6-O-methylerythromycin A (clarithromycin), shown below, is a potent macrolide antibiotic (United States Patent No. 4,331,803).

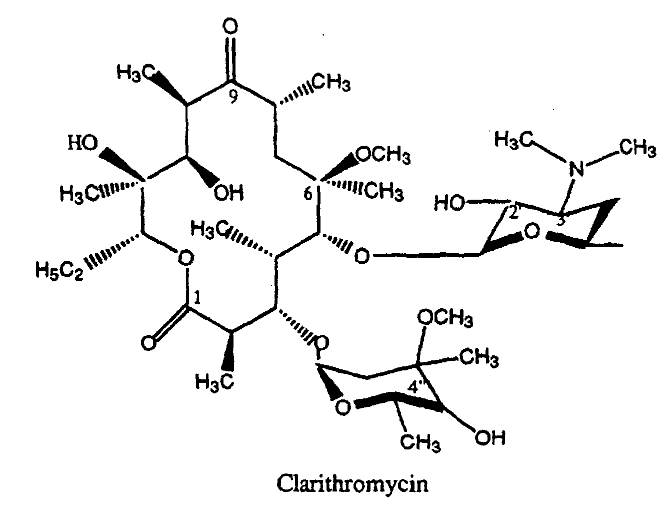

A variety of processes for preparing 6-O-methylerythromycin A have been described. By way of example, 6-O-methylerythromycin A can also be made from 9-oxime erythromycin A derivatives (See, e.g., United States Patent Nos. 5,274,085; 4,680386; 4,668776; 4,670,549, 3,478,014 and 4,672,109 and European Patent Application 0260938 A2).

When 9-oxime erythromycin A derivatives are utilized, the oxime can be made by reacting erythromycin A with either hydroxylamine hydrochloride and a base or free hydroxylamine in methanol and an organic add (see, e.g., United States Patent Nos. 3,478,014 and 5,274,085). The existing methods of making 9-oxime erythromycin A result in the production of degradation impurities.

There continues to be a need in the art for a method of making 9-oxime erythromycin A that minimizes degradation impurities while maintaining a high production yield.

### Brief Summary Of The Invention

The present invention provides a new and improved process of preparing 9-oxime erythromycin A. That process used a mild acid catalyst in combination with a mildly polar solvent.

In accordance with the present process, erythromycin A is reacted with hydroxylamine in the presence of acetic acid and isopropanol. In one embodiment, aqueous hydroxylamine is added to a solution of erythromycin A in isopropanol to form a reaction mixture and then acetic acid is added to the reaction mixture. In another embodiment, aqueous hydroxylamine is mixed with acetic acid to form a mixture and the mixture is then added to a solution of erythromycin A in isopropanol.

The use of isopropyl alcohol provides a homogenous reaction mixture. The use of acetic acid gives less degradation impurities. Advantages of the process of the present invention include a significant improvement in yield, ease of liquid reagent handling, the ready availability of the starting materials, reagents and solvent, and the direct recycling of the process solvents into the later stage of antibiotic (clarithromycin) synthesis.

### Detailed Description Of The Invention

The preparation of 9-oxime erythromycin A begins with erythromycin A which is dissolved in isopropanol. An aqueous solution of hydroxylamine is added to the erythromycin/isopropanol to form a reaction mixture. Acetic add is then added to the reaction mixture.

The reaction mixture is maintained at a suitable temperature for a period of time sufficient for 9-oxime formation. Temperature can range from about 35°C to about 65°C. Preferably, the temperature is from about 45°C to about 55°C and, more preferably about 50°C.

Following completion of 9-oxime formation, the reaction mixture is cooled to about room temperature. Isopropyl acetate is added to the cooled reaction mixture and the reaction mixture stirred. During stirring, the reaction mixture is titrated to a pH value of > 11.0 with sodium hydroxide. The organic layer is washed with a dilute caustic and concentrated to dryness to give 9-oxime erythromycin A.

In another embodiment, the acetic acid can be pre-mixed with the hydroxylamine free base prior to mixing it with erythromycin A. This allows for reduction of the exotherm upon erythromycin A addition.

As shown below in the Examples, the use of isopropanol and acetic add results in greater yields of the 9-oxime when compared to the use of methanol and acetic or formic acid.

9-Oxime erythromycin A prepared in accordance with a process of the present invention can then be used to prepare 6-O-methylerythromycin A in accordance with standard procedures well known in the art.

Those subsequent steps include the steps of protecting the 9-oxime group as well as the 2'-hydroxy group (with or without optional protection of the 3'-dimethylamine and 4"-hydroxy group), selective methylation of the 6-hydroxy group, deprotection of the protected groups and deoximation. Those steps can be carried out by a variety of means known in the art (See, e.g., U.S. Patent Nos. 5,274,085; 4,680,386; 4,668,776; 4,670,549 and 4,672,109 and European Patent Application 0260938 A2).

The following Examples illustrate preferred embodiments of the present invention and are not limiting of the specification and claims in any way.

### Example 1

### Production of 9-Oxime Erythromycin A

50% aqueous hydroxylamine (42.5 g.) was added to a stirred mixture of *Ery A* (50 g) in isopropanol (100 ml). To this mixture acetic acid (16.3 g) was added. A clear solution was formed and heated at 50°C until the completion of the reaction. At the end of the reaction, the mixture was cooled to room temperature, and isopropyl acetate (150 ml) was added. The mixture was vigorously stirred while 4N NaOH was added to adjust the pH to no less than 11.0. The organic layer was washed with dilute caustic and concentrated to dryness to give *Ery A* oxime. Alternatively, the organic layer was assayed to show a 98.9% yield.

### Example 2

### Comparison of the Use of Acetic Add/Isopropanol with Other Acid/Solvent Systems

9-Oxime erythromycin A was prepared in accordance with the procedures of Example 1 except that various combinations of acids and alcohols were used in addition to acetic acid and isopropanol. Those other combinations included methanol (MeOH) with formic acid (HCOOH), isopropanol (IPA) with HCOOH, MeOH with acetic add (HOAc), IPA with HOAc and ethanol (EtOH) with HOAc. The results of those studies are summarized below in Table 1.

**TABLE 1**

| **Solvent** | **Acid Catalyst** | **% Yield** |
|---|---|---|
| MeOH | HCOOH | 90.7 |
| MeOH | HCOOH | 92.9 |
| IPA | HCOOH | 91.0 |
| IPA | HCOOH | 88.5 |
| MeOH | HOAc | 93.6 |
| **IPA** | **HOAc** | **99.0** |
| **IPA** | **HOAc** | **97.6** |
| IPA | HOAc | 94.4 |
| EtOH | HOAc | 98.7 |

It can be seen from the data in Table 1 that the combination of isopropanol and acetic acid gave the optimum yields.

## Claims

1. A process of preparing 9-oxime erythromycin A comprising reacting erythromycin A with hydroxylamine in the presence of acetic acid and isopropanol.

2. The process of claim 1 wherein aqueous hydroxylamine is added to a solution of erythromycin A in isopropanol to form a reaction mixture and then acetic add is added to the reaction mixture.

3. The process of claim 1 wherein aqueous hydroxylamine is mixed with acetic acid to form a mixture and the mixture is then added to a solution of erythromycin A in isopropanol.

4. A process of preparing 6-O-methylerythromycin A comprising selectively methylating a 9-oxime erythromycin A prepared by the process of claim 1 to form a 6-O-methyl, 9-oxime erythromycin A and deoximating the formed 6-O-methyl, 9-oxime erythromycin A.

## Patentansprüche

1. Ein Verfahren zur Herstellung von 9-Oxim-Erythromycin A, das das Reagieren von Erythromycin A mit Hydroxylamin in der Anwesenheit von Essigsäure und Isopropanol umfaßt.

2. Das Verfahren gemäß Anspruch 1, worin wässeriges Hydroxylamin zu einer Lösung von Erythromycin A in Isopropanol hinzugefügt wird, um eine Reaktionsmischung zu bilden, und dann Essigsäure zu der Reaktionsmischung hinzugefügt wird.

3. Das Verfahren gemäß Anspruch 1, worin wässeriges Hydroxylamin mit Essigsäure gemischt wird, um eine Mischung zu bilden, und die Mischung dann zu einer Lösung von Erythromycin A in Isopropanol hinzugefügt wird.

4. Ein Verfahren zur Herstellung von 6-0-Methylerythromycin A, das die selektive Methylierung eines 9-Oxim-Erythromycin A, welches durch das Verfahren von Anspruch 1 hergestellt wurde, um ein 6-0-Methyl, 9-Oxim-Erythromycin A zu bilden, und die Deoximierung des gebildeten 6-0-Methyl, 9-Oxim-Erythromycin A, umfaßt.

## Revendications

1. Procédé pour la préparation de la 9-oxime de l'érythromycine A comprenant la réaction de l'érythromycine A avec l'hydroxylamine en présence d'acide acétique et d'isopropanol.

2. Procédé selon la revendication 1, dans lequel on ajoute de l'hydroxylamine aqueuse à une solution d'érythromycine A dans l'isopropanol pour former un mélange réactionnel, puis on ajoute de l'acide acétique au mélange réactionnel.

3. Procédé selon la revendication 1, dans lequel on mélange de l'hydroxylamine aqueuse à de l'acide acétique pour former un mélange, puis on ajoute le mélange à une solution d'érythromycine A dans l'isopropanol.

4. Procédé pour la préparation de la 6-O-méthylérythromycine A comprenant la méthylation sélective d'une 9-oxime de l'érythromycine A préparée par le procédé selon la revendication 1 pour former la 9-oxime de la 6-O-méthylérythromycine A, puis la désoximation de la 9-oxime de la 6-O-méthylérythromycine A formée.
